# EUROPÄISCHE PATENTANMELDUNG

(11) **EP 2 422 730 A1**
(43) Veröffentlichungstag der Anmeldung: **29.02.2012**
(21) Anmeldenummer: 11178272.8
(22) Anmeldetag: 22.08.2011
(51) Int. Cl.: A61B 17/70, A61B 17/88, A61M 29/00

(54) **Vorrichtung zur Ballon-Wirbelkyphoplastie**

(30) Priorität: 23.08.2010 RU 2010134842
(71) Anmelder: Korsakov, Konstantin Vladimirovich, Moskau 125167 (RU); Vilkov, Dmitry Yurievich, Samara 443087 (RU); Dubrovin, Andrey Evgenyevich, Moskovskaya oblast 142771 (RU); Toma, Aleksandr Ilyich, Saratov 413056 (RU)
(72) Erfinder: Toma, Aleksandr Ilyich, 410056 Saratov (RU); Elkin, Vladimir Aleksandrovich, 410031 Saratov (RU); Norkin, Aleksey Igorevich, 410031 Saratov (RU); Vilkov, Dmitry Yurievich, 410002 Saratov (RU); Toma, Ilya Aleksandrovich, 410056 Saratov (RU); Yankin, Sergey Sergeyevich, 410039 Saratov (RU)
(74) Vertreter: Sloboshanin, Sergej

(57) **Zusammenfassung**

Die Anmeldung betrifft die Medizintechnik, insbesondere Instrumente für die Traumatologie, Neurochirurgie und Vertebrologie, und kann zur Rekonstruktion der Wirbelsäule bei einem chirurgischen Eingriffsverfahren mit der Technik der Ballon-Kyphoplastie verwendet werden. Die Vorrichtung zur Ballon-Wirbelkyphoplastie umfasst eine Einrichtung zur Injektion von Knochenersatzmaterial in den Wirbelkörper, eine tragende Katheterröhre (1) mit einem Kanal zum Pumpen einer Druckflüssigkeit in einen abdichtend befestigten Dilatationsballon (3) aus elastischem, dehnbarem Material. Über dem Ballon ist eine Schutzhülle (4) angeordnet, die aus einem medizinischen Material mit Zellen, die von sich kreuzenden Fäden (6) gebildet werden, hergestellt ist. An der äußeren Oberfläche der Schutzhülle an den Fadenkreuzungspunkten (7) sind Fixierelemente (8) in Form langgestreckter Elemente angeordnet. Sie sind aus medizinischem Metall hergestellt und nach außen gerichtet, so dass sie in das Knochengewebe des Wirbels eindringen können, um die Schutzhülle darin zu befestigen.

## Beschreibung

Die Erfindung betrifft die Medizintechnik, insbesondere Instrumente für die Traumatologie, Neurochirurgie und Vertebrologie, und kann zur Rekonstruktion der Wirbelsäule bei einem chirurgischen Eingriffsverfahren mit der Technik der Ballon-Kyphoplastie verwendet werden, das auf der Grundlage der Punktions-Vertebroplastie zur Behandlung von Patienten mit Kompressionsbrüchen oder Kompressions-Splitterbrüchen der Wirbelkörper infolge von Osteoporose oder traumatischen Schädigungen entwickelt wurde und das es ermöglicht, die infolge der kyphotischen Deformation verlorene Höhe des Wirbelkörpers wiederherzustellen und diesen mit Hilfe einer Injektion von Knochenersatzmaterial in den Wirbelkörper zu verstärken [E.G. Pedachenko, S.V. Kushchaev: Punktsionnaya vertebroplastika, A.L.D., Kiew 2005, S. 25].

Es ist eine Einrichtung zur Durchführung einer Punktions-Vertebroplastie zur chirurgischen Behandlung von Kompressionsbrüchen und aggressiven Hämangiomen der Wirbelkörper bekannt.

[E.G. Pedachenko, S.V. Kushchaev: Punktsionnaya vertebroplastika, A.L.D., Kiew 2005, S. 517; H. Deramond, C. Depriester, P. Galibert, D. Le Gars: Percutaneous vertebroplasty with polymethyl methacrylate. Technique, indications, and results. Radiol Clin North AM, 1998, 36:533-546; J.M. Mathis, J.D. Barr et al: Percutaneous vertebroplasty: a developing standard of care for vertebral compression fractures. [Review.] AJNR, 2001; 22:373-381.]

Sie besteht aus einem Trokar, der unter fluoroskopischer oder computertomografischer Kontrolle zur Injektion von Knochenersatzmaterial zur Verstärkung des geschädigten Wirbelkörpers in den Wirbelkörper eingeführt wird.

Für Patienten mit Osteoporose und schweren traumatischen Schädigungen ist eine ausgeprägte kyphotische Deformation der Wirbelsäule charakteristisch, die zu einer Störung der Funktion der Lungen, einer Verringerung des Gesamtvolumens des Peritonealraums und ständigem Schmerz führt.

Jedoch war bei der Punktions-Vertebroplastie dieser Art kein konstruktives Element vorgesehen, das eine Korrektur der oben beschriebenen Deformation ermöglicht.

Zur Lösung dieses Problems wurde eine neue Art von Einrichtungen entwickelt, nämlich Vorrichtungen zur Kyphoplastie, die zusätzliche Elemente zur Beseitigung der Deformation und zur Wiederherstellung der Höhe des geschädigten Wirbelkörpers durch Ausbildung eines Hohlraums in diesem umfasst.

Es ist ebenfalls eine "Einrichtung zur Kyphoplastie" bekannt [Patent RU 2311149], das aus einer tragenden Katheterröhre besteht, an deren distalem Ende eine rechteckige Fläche ausgebildet ist, die innerhalb des Wirbelkörpers mechanisch ausfahrbar ist, um dessen Höhe wiederherzustellen.

Der Nachteil dieser Einrichtung ist das hohe Risiko einer Verkeilung der rechteckigen Fläche, wenn Knochengewebe unter sie gerät. Wegen der möglichen Verkeilung ist eine Entfernung der Einrichtung aus dem Wirbelkörper traumatisch.

Es sind auch Lösungen bekannt, die in dem Patent RU 2351375 und dem Gebrauchsmuster RU 71534 derselben Anmelderinnen sowie in den Patenten US 5972015 und 6899719 offenbart sind. Darin sind Einrichtungen zur Ballon-Kyphoplastie beschrieben, die aus einer tragenden Katheterröhre mit einem am distalen Ende befestigten Dilatationsballon aus elastischem Material bestehen. Zur Korrektur der kyphotischen Deformation kann der Dilatationsballon sich im Wirbelkörper unter Einwirkung einer Pumpflüssigkeit ausdehnen. Zur Injektion des Knochenersatzmaterials wird ein Trokarinjektor verwendet.

Jedoch ist bei diesen Einrichtungen kein Schutz des Dilatationsballons vor Einstichen durch scharfe Knochensplitter beim Ausdehnen des Ballons vorgesehen. Außerdem ist bei ihnen die Gefahr groß, dass das Knochersatzmaterial über die Grenzen des Wirbels hinaus austritt.

Es sind ebenfalls Vorrichtungen zur Kyphoplastie bekannt [Patente US 5549679, US 5571189, Patent EP 1408888], die eine Einrichtung zur Korrektur der Deformation des Wirbelkörpers, die die Schaffung eines Hohlraums in dessen Inneren ermöglicht, sowie eine tragende Katheterröhre mit einer am distalen Ende angeordneten Gitterhülle umfassen. Die tragende Katheterröhre kann den ausgebildeten Hohlraum mit Knochenersatzmaterial füllen und die Gitterhülle ausdehnen, die einen Durchtritt des Knochenersatzmaterials über die Grenzen des zuvor ausgebildeten Hohlraums verhindert.

Jedoch erfordern die bei dieser Einrichtung vorgesehene Ausdehnung und die Installation der Gitterhülle an den Grenzen des ausgebildeten Hohlraums die Injektion des Knochenersatzmaterials unter ausreichend hohem Druck, bei dem es schwierig ist, dessen gleichmäßige Verteilung zu erzielen und die Gefahr zu vermeiden, dass im Wirbelkörper Bereiche entstehen, die nicht mit diesem Material verfüllt sind.

Der zu der Erfindung nächstgelegene Stand der Technik ist eine Einrichtung zur Ballon-Kyphoplastie [Patent US 7226481], die Folgendes umfasst: Eine Einrichtung zur Injektion von Knochenersatzmaterial in den Wirbelkörper, eine tragende Katheterröhre mit einem Kanal zum Pumpen einer Druckflüssigkeit in einen an ihrem distalen Ende befestigten elastischen Dilatationsballon mit einer darüber angeordneten Schutzhülle, die aus einem dichten medizinischen Material besteht, beispielsweise Gewebe oder Metall. Der Ballon ist so ausgebildet, dass er die Höhe des Wirbelkörpers wiederherstellen kann, und so dass mit seiner Hilfe die Schutzhülle, die die Funktion einer Barriere erfüllt, die den Austritt von Knochenersatzmaterial über die Grenzen des Wirbels hinaus begrenzt, in dem ausgebildeten Hohlraum installiert werden kann.

Jedoch ermöglichen die konstruktiven Besonderheiten der Schutzhülle nicht, sie vor der bevorstehenden Injektion von Knochenersatzmaterial zuverlässig und fest in dem Knochengewebe zu befestigen. Dabei ist ein Einfallen der Wände des ausgebildeten Hohlraums möglich, das zu einem vollständigen oder teilweisen Verlust der Korrektur der Form des geschädigten Wirbels führt, beispielsweise aufgrund eines Abbrechens von Knochensplittern. Außerdem kann bei einem Fehlen einer zuverlässigen Befestigung an den Rändern am Umfang des ausgebildeten Hohlraums die Schutzhülle zusammenfalten und es kann unmöglich werden, sie mit Hilfe des Knochenersatzmaterials zu richten, wobei es, wie oben erwähnt, schwierig ist, dessen gleichmäßige Verteilung zu ermöglichen und die Gefahr zu vermeiden, dass in dem Wirbelkörper Bereiche entstehen, die nicht mit diesem Material verfüllt sind.

Aufgabe der Erfindung ist die Entwicklung einer Vorrichtung zur Ballon-Wirbelkyphoplastie, die eine stabile Position des gerichteten und mit Hilfe des Dilatationsballons wiederhergestellten Wirbelkörpers gewährleistet, auch im Moment des Entleerens des Ballons vor der Injektion des Knochenersatzmaterials.

Der Kern der Erfindung besteht darin, dass in eine Vorrichtung zur Ballon-Wirbelkyphoplastie, die Folgendes umfasst: Eine Einrichtung zur Injektion von Knochenersatzmaterial in den Wirbelkörper, eine tragende Katheterröhre mit einem Kanal zum Pumpen von Druckflüssigkeit in einen am distalen Ende der tragenden Katheterröhre abdichtend befestigten Dilatationsballon aus elastischem, dehnbarem Material mit einer darüber angeordneten Schutzhülle in entsprechender Größe, die aus einem medizinischen Material mit Zellen besteht, die aus sich kreuzenden Fäden bestehen, *an der äußeren Oberfläche der Schutzhülle* an den Fadenkreuzungspunkten Fixierelemente in Form langgestreckter Elemente angeordnet sind, die aus medizinischem Metall bestehen und nach außen gerichtet sind, so dass sie in das Knochengewebe des Wirbels eindringen können, um die Schutzhülle darin zu befestigen.

Außerdem wird eine Vorrichtung zur Ballon-Wirbelkyphoplastie mit den oben genannten Merkmalen angemeldet, *bei der die Fixierelemente in einer Anzahl vorhanden sind, die geringer als die Anzahl der Fadenkreuzungspunkte ist.*

Es wird weiterhin eine Vorrichtung zur Ballon-Wirbelkyphoplastie mit den oben genannten Merkmalen angemeldet, *bei der der Dilatationsballon zusätzlich eine und*/*oder mehrere Kammern aufweist.*

### Technisches Ergebnis der Erfindung

Die Verwendung der Fixierelemente, die an der Schutzhülle angeordnet und von der Hülle nach außen ausgerichtet sind, vereinfacht die Anordnung der Hülle in dem ausgebildeten Hohlraum durch die feste Fixierung in dem durch den Dilatationsballon verdichteten schwammigen Knochengewebe. Dabei wird die Gefahr eines Einfallens der Schutzhülle ausgeschlossen. Bei ihrer Aufrichtung unter dem Druck der in den Dilatationsballon gepumpten Flüssigkeit dringen die Fixierelemente in Form langgestreckter Elemente aus festem medizinischen Metall tief in das Knochengewebe ein und werden zuverlässig darin fixiert.

Dadurch bildet die mit Hilfe der Vorrichtung installierte und fixierte Schutzhülle eine Karkasse, die Knochensplitter in dem ausgebildeten Hohlraum zurückhält, und gewährleistet eine stabile Position des aufgerichteten und wiederhergestellten Wirbelkörpers. Die Wahl der Fadenkreuzungspunkte als Anordnungsstellen für die Fixierelemente ist dadurch bedingt, dass sie eine wirksame Stütze für die langgestreckten Elemente sind, die unter Druck in das Knochengewebe eindringen. Ihre wirksamste Anordnung über die gesamte Oberfläche der Schutzhülle in ausreichender Anzahl, die in der Regel geringer ist als die Anzahl der Kreuzungspunkte, ermöglicht eine gleichmäßige Fixierung ohne Überlastung des Aufbaus der Schutzhülle. Dabei sind verschiedene Mechanismen zum Installieren der Fixierelemente möglich, beispielsweise Mikroschweißen oder Einspleißen von in einem spitzen Winkel umgebogenen Metallfäden in das Material der Schutzhülle, wodurch Fixierelemente in Form von Elementen gebildet werden, die aus der Hülle nach außen vorstehen.

Die Verwendung eines Dilatationsballons, der zusätzlich eine und/oder mehrere Kammern aufweist, ermöglicht eine Erhöhung der Wirksamkeit bei schrittweiser Wiederherstellung der Höhe des geschädigten Wirbelkörpers, wobei die unteren Kammern eine Art "Sockel" für die oberen Kammern bilden.

Die Erfindung wird anhand von Fig. 1 bis 4 erläutert, die Folgendes darstellen:
- Fig. 1: die Vorrichtung zur Ballon-Wirbelkyphoplastie;
- Fig. 2: einen Teil der Schutzhülle;
- Fig. 3: einen Wirbelkörper mit Kompressionsbruch;
- Fig. 4: die Wiederherstellung der Höhe des Wirbelkörpers mit der Vorrichtung zur BallonWirbelkyphoplastie.

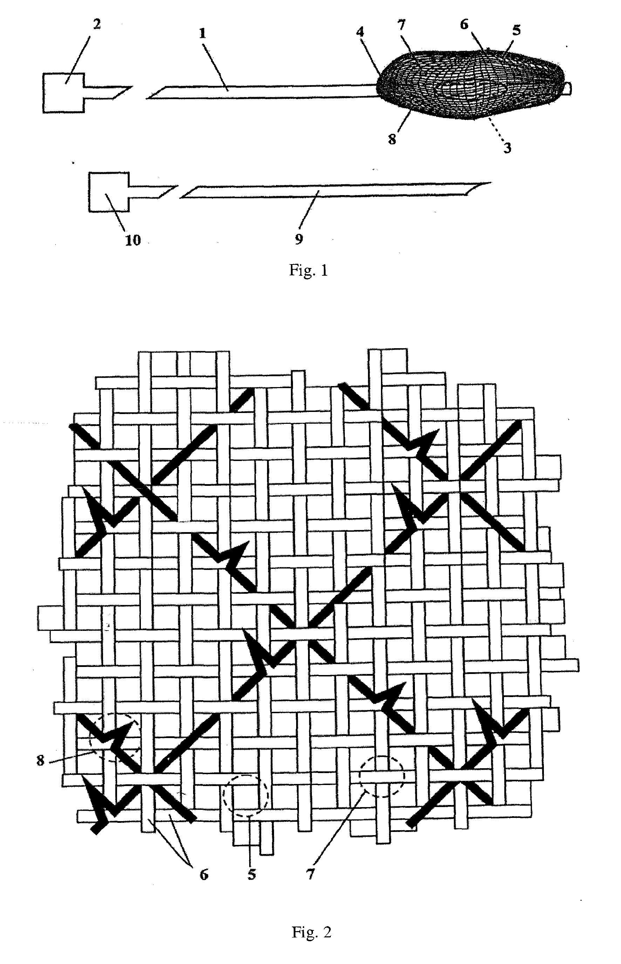

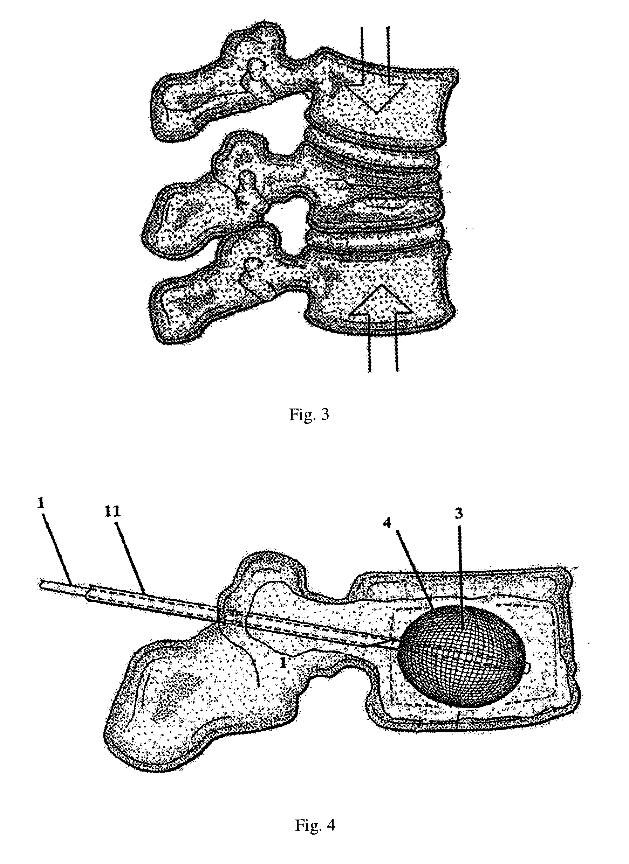

In Fig. 1 bis 4 sind mit den Bezugszeichen 1 bis 11 bezeichnet:
- 1: die tragende Katheterröhre,
- 2: die Vorrichtung zum Pumpen von Druckflüssigkeit,
- 3: der Dilatationsballon,
- 4: die Schutzhülle,
- 5: Zellen der Schutzhülle,
- 6: die sich kreuzenden Fäden,
- 7: die Fadenkreuzungspunkte,
- 8: die Fixierelemente,
- 9: die Nadel,
- 10: die Spritze mit dem Knochenersatzmaterial,
- 11: der Röhrenleiter.

Die Vorrichtung zur Ballon-Wirbelkyphoplastie umfasst eine tragende Katheterröhre 1 mit einem Kanal, an den die Einrichtung 2 zum Pumpen von Druckflüssigkeit angeschlossen ist.

Am distalen Ende der tragenden Katheterröhre ist der Dilatationsballon 3 mit der darüber angeordneten entsprechend großen Schutzhülle 4 abdichtend befestigt.

Als Dilatationsballon können Ballons aus Latex, Silikon oder einem beliebigen anderen elastischen, dehnbaren Material mit einem spezifischen Berstdruck von wenigstens 20 atm verwendet werden.

Die Schutzhülle 4 ist aus medizinischem Material, beispielsweise Kohlenstofffaser, Nitinol, Gore-Tex und Ähnlichem, mit sich kreuzenden Fäden 6, die Zellen 5 bilden, hergestellt.

Die Größe einer Seite der Zellen 5 liegt im Bereich von 100 bis 5000 µm. Bei Verwendung von Knochenzement als Knochenersatzmaterial wird eine Schutzhülle 4 mit Zellen 5 in einer Größe von nicht mehr als 250 µm gewählt. Bei diesen Parametern lässt die Schutzhülle 4 keine Zementpartikel durch, deren typische Größe bei mehr als 250 µm liegt, wodurch deren Austritt über die Grenzen des Wirbels hinaus verhindert wird, bei dem der Knochenzement eine zerstörerische Wirkung auf die Nervenenden und die weichen Gewebe des menschlichen Organismus ausüben kann. Bei Verwendung von Knochenspänen besteht keine Notwendigkeit, den Austritt des Knochenersatzmaterials zu verhindern, da Knochenspäne keine negative Auswirkung auf den Organismus haben. Die Schutzhülle 4 verhindert keine Strömungen von Blut bildenden Elementen durch den geschädigten Wirbel.

An der äußeren Oberfläche der Schutzhülle 4 sind an den Fadenkreuzungspunkten 7 Fixierelemente 8 angeordnet, die durch Punktschweißen an den Fadenkreuzungspunkten 7 oder durch Aufspleißen der Metallfäden in der Schutzhülle 4 befestigt werden können. Falls Metallfäden angewendet werden, sind die Fixierelemente 8 aus in einem spitzen Winkel gebogenen Elementen dieser Fäden ausgebildet.

Die Fixierelemente sind aus medizinischem Metall hergestellt, beispielsweise aus Wolfram, Titan oder Tantal in Form von langgestreckten Elementen, die von der Schutzhülle 4 nach außen gerichtet sind. Die Länge der Fixierelemente 8 liegt im Bereich von 500 bis 1000 µm, was es ihnen ermöglicht, wenn sie aus den oben genannten Materialien hergestellt sind, in das Knochengewebe einzudringen, um die Schutzhülle 4 dort festzuhalten und dadurch die Wände des in dem Wirbelkörper ausgebildeten Hohlraums zu befestigen.

Die Vorrichtung zur Ballon-Wirbelkyphoplastie umfasst eine Einrichtung zur Injektion von Knochenersatzmaterial in den Wirbelkörper, die aus einer Nadel 9 und einer Spritze 10 mit dem Knochenersatzmaterial besteht.

Eine Verwendung eines Dilatationsballons 3, der zusätzlich eine und/oder mehrere Kammern aufweist, ist möglich, was eine Erhöhung der Wirksamkeit bei einer schrittweisen Wiederherstellung der Höhe des geschädigten Wirbelkörpers ermöglicht, wobei die unteren Kammern eine Art "Sockel" für die oberen Kammern bilden.

Die Vorrichtung funktioniert wie folgt.

Der Patient wird in Bauchlage auf den Operationstisch gelegt. Der Operationsbereich wird markiert, wo Orientierungspunkte zur Durchführung einer transpedikulären Punktion des geschädigten Wirbels angeordnet werden, die mittels Einführung eines Röhrenleiters 11 zur Einführung der angemeldeten Vorrichtung durchgeführt wird, kontrolliert durch Röntgen und/oder Computertomografie und unter Lokalanästhesie. Durch den Röhrenleiter 11 wird in einem in dem Wirbelkörper ausgebildeten Kanal der distale Teil der tragenden Katheterröhre 1 installiert, an dem am distalen Ende der Dilatationsballon 3 abdichtend, beispielsweise durch abdichtende Nähte, befestigt wird.

Durch die Ausdehnung des Dilatationsballons 3 mit der darüber aufgezogenen, entsprechend großen Schutzhülle 4 wird unter Einwirkung der Flüssigkeit, die von der Einrichtung 2 zum Pumpen der Druckflüssigkeit gepumpt wird, ein Hohlraum in dem geschädigten Wirbelkörper gebildet, wodurch seine Form wiederhergestellt und die kyphotische Deformation korrigiert wird.

Bei der Ausdehnung des Dilatationsballons 3 werden die Zellen 5 der Schutzhülle ausgedehnt, die durch die sich kreuzenden Fäden 6 gebildet werden; die Fixierelemente 8, die an den Fadenkreuzungspunkten 7 angeordnet sind, dringen fest in das verdichtete Knochengewebe ein und befestigen die Schutzhülle 4 in dem ausgebildeten Hohlraum. Die fixierte Schutzhülle 4 bildet eine Karkasse, die Knochensplitter zurückhält, die infolge des Bruchs auftreten und sich an den Wänden des ausgebildeten Hohlraums befinden.

Nach der Installation der Schutzhülle 4 wird der Dilatationsballon 3 entleert und zusammen mit der tragenden Katheterröhre 1 aus dem ausgebildeten Hohlraum entfernt. Anschließend wird die Einrichtung zur Injektion des Knochenersatzmaterials in den Wirbelkörper in diesen Hohlraum eingeführt, die aus der Nadel 9 besteht, durch deren Öffnung am distalen Ende die Verfüllung des Hohlraums mit Knochenersatzmaterial durchgeführt wird, das durch die Spritze 10 zugeführt wird.

Anschließend werden alle Elemente und Einheiten der Vorrichtung zur Ballon-Wirbelkyphoplastie entfernt und ein aseptisches Pflaster wird auf die Punktionsstelle geklebt. Das Knochenersatzmaterial härtet aus und ermöglicht die Tragfähigkeit des geschädigten Wirbels.

### Beispiel

Patient M., 56 Jahre, kam in die traumatologische Abteilung mit der Diagnose:
"Geschlossenes einfaches Trauma im Brustwirbelbereich mit Kompressionsbruch des Wirbelkörpers ThXII."

Das Trauma hatte der Patient infolge eines Verkehrsunfalls erlitten. Vom Unfallort wurde der Patient 4 Stunden nach Erleiden des Traumas in das Krankenhaus eingeliefert.

An dem Patienten wurde eine röntgenologische und eine computertomografische Untersuchung durchgeführt, bei denen ein Kompressionsbruch des Wirbelkörpers ThXII mit einem Keilförmigkeitsindex von 0,65 und einem kyphotischen Deformationswinkel von 19° festgestellt wurde, eine Verschiebung von Knochenfragmenten zum Wirbelkanal wurde nicht festgestellt. Bezüglich des neurologischen Status wurden keine motorischen und sensorischen Ausfallerscheinungen festgestellt. Lokal wurde bei Palpation eine ausgeprägte Empfindlichkeit der Dornfortsätze auf der Höhe ThXI-LI festgestellt. Der Patient wurde in eine reklinierende Hängematte mit Gewichten von je 8 kg auf jeder Seite gelegt. Es wurde eine konservative symptomatische Therapie bestimmt. Nach drei Tagen wurde an dem Patienten eine Röntgenkontrolle durchgeführt, die ergab, dass der Grad der Kompression des Wirbelkörpers ThXII praktisch auf demselben Stand geblieben war.

Am vierten Tag nach Erleiden des Traumas wurde an dem Patienten eine chirurgische Behandlung des Wirbelkörpers ThXII unter Verwendung der Vorrichtung zur Ballon-Kyphoplastie vorgenommen. Bei der Operation wurde der Patient in Bauchlage mit Walzen unter Brustkorb und Becken auf den Operationstisch gelegt. Es wurde die übliche Vorbereitung des Operationsbereichs von der Höhe der Schulter bis zum Kreuzbein durchgeführt und eine Lokalanästhesie mit einer 1%-igen Lidocain-Lösung schichtweise entsprechend dem vorgesehenen Punktionspfad am Vorsprung der Bogenwurzel des Wirbels ThXII links vorgenommen. Weiterhin wurde der Röhrenleiter unter fluoroskopischer Kontrolle zur Bogenwurzel des Wirbels ThXII eingeführt und positioniert, anschließend wurde der Leiter bis zur Grenze des vorderen und des mittleren Drittels des Wirbelkörpers (2/3 des Abstands von der Rückwand und 1/3 von der Vorderwand des Wirbelkörpers) in Richtung zur Mittellinie zu dem Bereich der stärksten Kompression des Wirbelkörpers bewegt. Durch den Röhrenleiter wurde die tragende Katheterröhre mit dem am distalen Ende angeordneten, abdichtend befestigten Dilatationsballon in den Wirbelkörper ThXII eingeführt.

Anschließend wurde eine Wiederherstellung der Höhe des Wirbelkörpers auf Normalmaß durch Ausdehnung des Dilatationsballons durchgeführt, dabei wurde eine Schutzhülle aus Kevlar mit Fixierelementen aus Wolfram in dem ausgebildeten Hohlraum angeordnet. Dabei wurde eine feste Fixierung der Schutzhülle in dem verdichteten Knochengewebe festgestellt. Der Dilatationsballon wurde entleert und zusammen mit der tragenden Katheterröhre aus dem Wirbelkörper entfernt. Durch den Röhrenleiter wurde die Einrichtung zur Injektion des Knochenersatzmaterials in den Wirbelkörper ThXII eingeführt und der ausgebildete Hohlraum im Wirbelkörper wurde mit Knochenersatzmaterial mit einem Volumen von 2 cm³ in Form von kleinem Granulat verfüllt, das mit Eigenblut und Omnipack-Kontrastmittel gemischt war. Dabei wurde kein Austritt des Knochenersatzmaterials über die Grenzen des Wirbelkörpers hinaus beobachtet.

Bei der Röntgenkontrolle wurde eine dichte Füllung des Hohlraums mit Knochenersatzmaterial festgestellt. Anschließend wurden die Einrichtung zur Injektion von Knochenersatzmaterial und der Röhrenleiter entfernt und ein aseptisches Pflaster wurde auf die Punktionsstelle aufgelegt. Am folgenden Tag wurde der Patient in eine vertikale Position gebracht. Kein schmerzhaftes Syndrom war vorhanden. Auf den Kontroll-Röntgenaufnahmen wurde eine vollständige Wiederherstellung der Höhe des Wirbelkörpers ThXII und eine Korrektur der kyphotischen Deformation festgestellt. Der Patient erhielt ein halbstarres Korsett und wurde zur ambulanten Behandlung entlassen. Bei einer wiederholten Röntgenuntersuchung nach 6 Monaten wurde kein Verlust der Korrektur festgestellt.

## Patentansprüche

1. Vorrichtung zur Ballon-Wirbelkyphoplastie, die Folgendes umfasst: Eine Einrichtung zur Injektion von Knochenersatzmaterial in den Wirbelkörper, eine tragende Katheterröhre mit einem Kanal zum Pumpen von Druckflüssigkeit in einen am distalen Ende der tragenden Katheterröhre abdichtend befestigten Dilatationsballon aus elastischem, dehnbarem Material mit einer darüber angeordneten Schutzhülle in entsprechender Größe, die aus einem medizinischen Material mit Zellen besteht, die aus sich kreuzenden Fäden bestehen, ***dadurch gekennzeichnet,* dass** an der äußeren Oberfläche der Schutzhülle an den Fadenkreuzungspunkten Fixierelemente in Form langgestreckter Elemente angeordnet sind, die aus medizinischem Metall hergestellt und nach außen gerichtet sind, so dass sie in das Knochengewebe des Wirbels eindringen können, um die Schutzhülle darin zu befestigen.

2. Vorrichtung zur Ballon-Wirbelkyphoplastie nach Anspruch 1, ***dadurch gekennzeichnet,* dass** die Anzahl der Fixierelemente geringer ist als die Anzahl der Fadenkreuzungspunkte.

3. Vorrichtung zur Ballon-Wirbelkyphoplastie nach Anspruch 1, ***dadurch gekennzeichnet,* dass** der Dilatationsballon zusätzlich eine und/oder mehrere Kammern aufweist.
